# EUROPEAN PATENT APPLICATION

(11) **EP 0 860 803 A2**
(43) Date of publication of application: **26.08.1998**
(21) Application number: 98301371.5
(22) Date of filing: 25.02.1998
(51) Int. Cl.: G08B 21/00

(54) **Carer's monitoring system**

(30) Priority: 25.02.1997 GB 9703892; 17.07.1997 GB 9714942
(71) Applicant: Lunan Products Limited, Arbroath DD11 3LR (GB)
(72) Inventor: Lamond, Margaret Butchart, Forfar Road, Arbroath, DD11 3RH (GB); Gall, Graeme, Dundee, DD4 0XQ (GB)
(74) Representative: Pacitti, Pierpaolo A.M.E.

(57) **Abstract**

This invention relates to a monitoring system for assisting a person who is responsible for the care of a child or adult requiring assistance when moving from one place to another. The system can inform the responsible person or carer if the person requiring assistance attempts to move on their own or has not been moved for a long period. The system includes (i) a carer unit comprising an alarm, a receiver and a display means for indicating the location of the person requiring assistance and (ii) a plurality of user units, each user unit comprising:
- a pressure sensing mat,
- a control unit coupled to the pressure mat, and
- a transmitter,
whereby transmitter transmits a trigger signal to the carer unit when pressure is removed from the pressure mat.

## Description

This invention relates to a monitoring system for assisting a person who is responsible for the care of a child or adult requiring assistance when moving from one place to another. In particular the monitoring system can inform the responsible person or carer if the person requiring assistance attempts to move on their own or has not been moved for a long period.

At present there does not exist a low cost product designed to help a carer in monitoring children or adults who require assistance when moving from one place to another. There is no simple method of monitoring "at risk" patients who require help moving from bed to chair, children who are prone to sleepwalking and require supervision, or patients who are prone to wander from their bed or chair.

It is an object of the invention to provide a low cost monitoring system which can be used to monitor one or more users and to inform a carer when a user leaves the bed or chair occupied by the user.

According to a first aspect of the present invention there is provided a monitoring system comprising:
- a pressure sensing mat having electrical contact surfaces which are brought into contact when sufficient pressure is applied to the mat,
- a control means coupled to said pressure mat, and
- an alarm unit
whereby the control means is adapted to transmit a first trigger signal to the alarm unit when pressure is removed from the pressure mat such that the electrical contact surfaces are separated from each other.

Preferably the control means includes delay means adapted to delay the transmission of the first trigger signal until the electrical contact surfaces are separated from each other for a first predetermined time, preferably of the order of 10 seconds or less.

Preferably the system further comprises a timing means adapted to measure the continuous elapsed period of time for which the electrical contact surfaces are in contact. Preferably the control means is adapted to transmit a second trigger signal when the elapsed period of time reaches a second predetermined time, preferably of between 0 and 6 hours, more preferably of the order of two or three hours.

Preferably the control means further comprises a time setting means adapted to permit selection of one or both of the first and second predetermined times.

Preferably the system further comprises a radio transmitter and a radio receiver. Preferably the trigger signal is a radio signal, most preferably a coded radio signal.

Preferably the pressure mat is flexible and is of the type for use with intruder alarms. Preferably the mat is less than 5 mm thick, most preferably approximately 3 mm thick.

Preferably the control means comprises a control unit having a switching means enabling the unit to be switched between an operative state and a non-operative state. Preferably the control unit is battery operated. The control unit may also have a manually operated alarm switch which when operated causes the control means to send a third trigger signal to the alarm unit. The control unit may further comprise a lamp.

Preferably the alarm unit comprises an audible alarm generating means such as a tone generator.

Alternatively the alarm unit comprises a visible alarm generating means such as a light. Preferably the alarm unit has a switching means enabling the unit to be switched between an operative state and a non-operative state. Preferably the alarm unit has a reset means enabling the alarm generating means to be switched off while leaving the alarm unit in an operative state. The alarm unit may be battery or mains operated.

The control unit may further comprise a position indication means. The position indication means preferably comprises at least one input device which may be operated to select an alphanumeric code. Preferably the alphanumeric code is transmitted as part of the coded signal sent to the receiver. Preferably the or each input device is a rotary dial which may be set to one of a plurality of positions, each position corresponding to an alphanumeric character. Preferably the alarm unit is provided with a display means for displaying the alphanumeric code associated with the coded signal.

According to a second aspect of the present invention there is provided a monitoring system comprising a carer unit and a plurality of user units, the carer unit comprising an alarm and a receiver, each user unit comprising:
- a pressure sensing mat having an electrical circuit means which changes from open to short circuit when sufficient pressure is applied to the mat,
- a control means coupled to said pressure mat, and
- a transmitter,
whereby the control means is adapted to transmit a first trigger signal to the alarm unit when pressure is removed from the pressure mat such that the electrical circuit means changes from short circuit to open circuit.

Preferably the control means includes delay means adapted to delay the transmission of the first trigger signal until the electrical contact surfaces are separated from each other for a first predetermined time, preferably of the order of 10 seconds or less.

Preferably each user unit further comprises a timing means adapted to measure the continuous elapsed period of time for which the electrical contact surfaces are in contact. Preferably the control means is adapted to transmit a second trigger signal when the elapsed period of time reaches a second predetermined time, selected according to the maximum time for which a user may safely remain in one position, preferably a period of up to six hours, more preferably of the order of two or three hours.

Preferably each user unit further comprises a time setting means adapted to permit selection of one or both of the first and second predetermined times.

Preferably the transmitter is a radio transmitter and the receiver is a radio receiver. Preferably the trigger signal is a radio signal, most preferably a coded radio signal.

Preferably the pressure mat is flexible and is of the type for use with intruder alarms. Preferably the mat is less than 5 mm thick, most preferably approximately 3 mm thick.

Preferably the user unit has a switching means enabling the unit to be switched between an operative state and a non-operative state. Preferably the user unit is battery operated. The user unit may also have a manually operated alarm switch which when operated causes the control means to send a third trigger signal to the carer unit.

Preferably the carer unit comprises one or more audible alarm generating means such as a tone generator. Alternatively the carer unit comprises one or more visible alarm generating means such as a light. Preferably the first and second trigger signals cause first and second alarm generating means to be triggered respectively. Preferably the carer unit has a switching means enabling the unit to be switched between an operative state and a non-operative state. Preferably the carer unit has a reset means enabling the alarm generating means to be switched off after being triggered by a trigger signal from a first user unit, while leaving the alarm unit in an operative state, whereby the alarm will operate on receiving a trigger signal from a second user unit. Preferably the carer unit has memory means for storing a trigger signal received from a second or subsequent user units while generating an alarm triggered by a trigger signal from a first user unit. The carer unit may be battery or mains operated.

Each user unit may further comprise a position indication means. The position indication means preferably comprises at least one input device which may be operated to select an alphanumeric code, which may correspond to the position in use of the user unit. Preferably the alphanumeric code is transmitted as part of the coded signal sent to the receiver. Preferably the or each input device is a rotary dial which may be set to one of a plurality of positions, each position corresponding to an alphanumeric character. Preferably the carer unit is provided with a display means for displaying the alphanumeric code associated with the coded signal which triggers the alarm. The display means may comprise one or more LCD or LED characters. Preferably the number of characters corresponds to the number of input devices on the user unit. Preferably the carer unit is provided with means for displaying the alphanumeric codes associated with a plurality of trigger signals stored in the memory means.

According to a third aspect of the present invention there is provided a monitoring system comprising:
- a pressure sensing mat having electrical contact surfaces which are brought into contact when sufficient pressure is applied to the mat,
- a control means coupled to said pressure mat,
- an alarm unit, and
- timing means adapted to measure the continuous elapsed period of time for which the electrical contact surfaces are in contact,
whereby the control means is adapted to transmit a trigger signal when the elapsed period of time reaches a predetermined time, preferably of between 0 and 6 hours, more preferably of the order of two or three hours.

Preferably the control means further comprises a time setting means adapted to permit selection of the predetermined time.

Embodiments of the invention will now be described, by way of example only, with reference to the accompanying figures, where:
Fig. 1 is a schematic view of a monitoring system according to the first aspect of the invention;
Fig. 2 is a schematic view of a monitoring system according to the second aspect of the invention;
Fig. 3 shows front, end and side views of a user unit or control unit of the monitoring system of Fig. 2;
Fig. 4 shows a front view of a carer unit or remote alarm of the monitoring system of Fig. 2;
Fig. 5 shows a circuit diagram for the user unit of Fig. 3;
Fig. 6 shows a circuit diagram for the carer unit base of Fig. 4; and
Fig. 7 shows a circuit diagram for the carer unit display of Fig. 4.

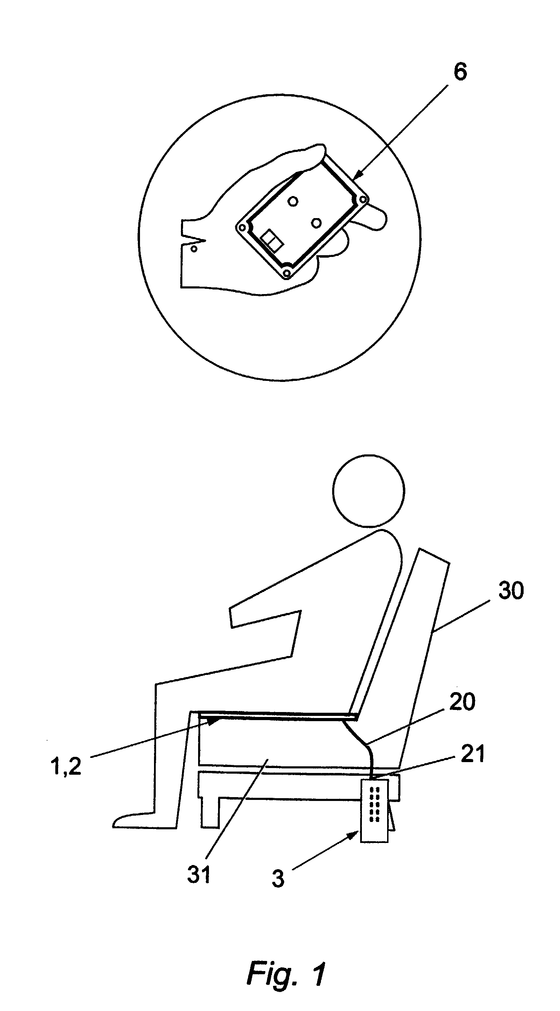

Referring to Fig. 1 there is shown a single user monitoring system comprising a pressure mat 1 which is encased in a hospital approved breathable fabric 2 which serves as a mat cover. The pressure mat 1 is of the sort commonly used in intruder detection systems and is thin and flexible. The mat gives an abrupt change from open to short circuit when a sufficiently large force is applied to it. In use the pressure mat 1 may be placed on or underneath the seat cushion 31 of a chair, and when the chair 30 is unoccupied the circuit is open, but when the chair 30 is occupied the circuit is closed. Alternatively the mat may be placed underneath a pillow, above, within or beneath a mattress or in a child's cot.

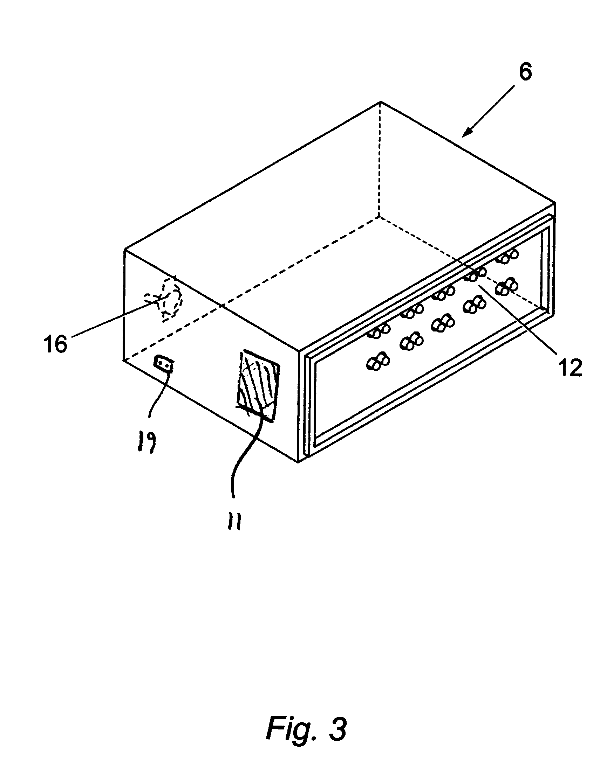

A cable 20 leads from the pressure mat 1 to the input socket 21 of a control unit or user unit 3. The user unit 3 is a hand held box, illustrated in detail in Fig. 3. The unit may have a fastening means such as a strap, bracket or hook and loop fastening material so that it can be readily attached to a chair or bed. The fastening means may be magnetic so that the unit can be attached to a metal bed or chair. The user unit incorporates a radio transmitter, and sends a radio signal when an open circuit is detected at the pressure mat 1. Although a delay may be incorporated, so that the radio signal is not sent when the circuit opens for a moment and then immediately closes again, as may occur when a patient or user shifts position, in practice it has been found that the unit functions effectively without a delay. A short alarm signal at the carer unit 13 is acceptable and indicates that the patient has momentarily moved, while a long or continuous alarm signal warns the carer that immediate action must be taken.

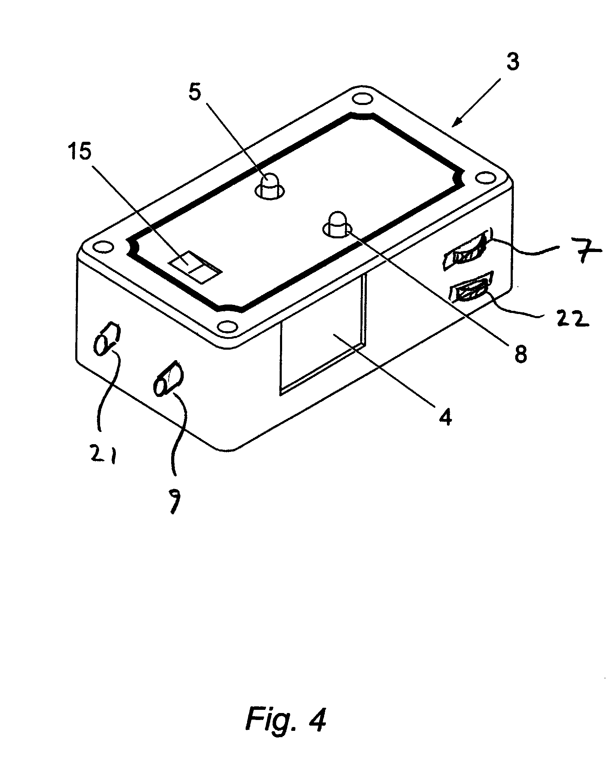

The radio signal is picked up by a receiver in the carer unit 13, which then operates an alarm, such as a warning sound. The carer unit 13 may also be in the form of a battery-operated hand held box, illustrated in detail in Fig. 4. The carer unit may have a strap or clip so that it may be worn by the carer.

Alternatively the carer unit may be mains operated and used in fixed locations.

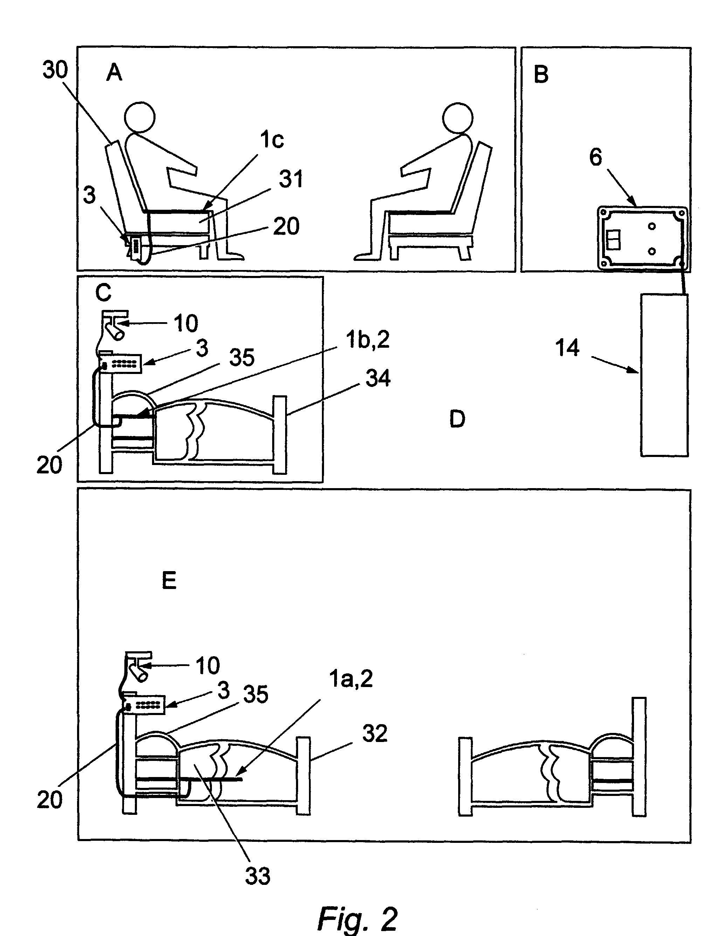

Referring now to Fig. 2, there is shown a multi-user monitoring system according to the invention. In the example illustrated the system has three users, but any number of users may be connected at one time. A first pressure mat 1A is placed beneath the mattress 33 of a bed 32 in a ward designated "Area 1". A second pressure mat 1B is placed beneath the pillow 35 of a bed 34 in a private room designated "Area 2". A third pressure mat 1C is placed on top of the seat cushion 31 of a seat 30 in a sitting room designated "Area 3". Each pressure mat 1A, 1B, 1C is connected by a cable 20 to a control or user unit 3, illustrated in Fig. 3, and operates in the same way as the pressure mat 1 of the single user system.

The user unit 3 has an on/off switch 15, which is switched on by the carer when the user is in position on the chair or bed, so that the user unit 3 is operative. A green power on pressure light 5 illuminates when the unit is operative. The unit is battery operated 4 and an optional red battery low light 8 illuminates when the battery voltage falls below a predetermined value. At the side of the user unit 3 is an input socket 21 for the cable 20 connected to the pressure mat 1. The unit may also have a medium to high voltage output 9 for a lamp 10 which illuminates when the alarm is triggered. This assists the carer at night time in seeing which bed in a ward has triggered the alarm. The lamp 10 can also assist the patient in the case of a fall or similar.

The user unit 3 also has a time setting dial 22. This can be set to an "off" position (position 0) or can be set to indicate a predetermined time in hours, corresponding to the period for which it is safe to leave a patient in one position without risk of developing pressure sores. If the dial 22 is set to a non-zero time, the user unit 3 sends a trigger signal to the carer unit 6 when the pressure mat is subject to a continuous pressure for more than the predetermined time. The carer then knows that it is time to move the patient. By moving the patient the carer re-sets the user unit, so that the user unit again starts to measure the time for which the pressure mat is subject to a continuous pressure.

The user unit 3 for a multi user system has an area indicator dial 7. This can be set to indicate a unique two-digit code for each user unit. Area indicator dials are not necessary for a single user system. In the system shown in Fig. 2 the two area indicator dials 7 of the user unit 3 connected to pressure mat 1A could be set to "0" and "1", indicating "Area 1", the two area indicator dials 7 of the user unit 3 connected to mat pressure 1B could be set to "0" and "2", indicating "Area 2", and so on. The radio signal sent by the transmitter in the user unit 3 when the circuit in the pressure mat 1 is opened is a coded radio signal. The coded signal includes a code representing the setting of the area indicator(s) 7 on the user unit 3.

Fig. 4 shows a carer unit 6 for use in a multi-user system. The carer unit 13 for a single user system is similar but does not have an LED display 12 and a wall display output 19. The carer unit 6 has an on/off switch 16 which is switched on by the carer when one or more user units 3 are switched on, so that the carer unit 6 is also operative. A green power on light 17 illuminates when the unit is operative. The unit may be battery operated or mains operated and an optional red battery low light 18 illuminates when the battery voltage falls below a predetermined value.

When a coded radio signal is sent by a transmitter in a user unit 3, the signal is received and decoded by the carer unit 6. This triggers the alarm, for example a warning tone emitted through a loudspeaker 11, and causes an LED display (not shown) to indicate the two-digit (or single digit) code representing the setting of the area indicator(s) 7 on the user unit 3 which has transmitted the coded radio signal, or to cause an appropriate light of the light pairs 12 to illuminate. Each light pair consists of a red light and a green light and corresponds to one of the codes or locations of the user unit. A red light indicates that an alarm has been triggered. A green light indicates that there is no alarm signal present. In this way, the carer is immediately informed of the location of the incident which has caused the alarm to be triggered.

A different alarm, for example a different tone, may be triggered depending on the type of signal. A signal indicating that the patient has left the mat 1 would have a more urgent alarm tone than a signal indicating that it is time to move the patient.

The carer unit 6 may have an output socket 19 for connection to a wall mounted display 14. This is appropriate when the carer unit is located in a room such as a nurses' station, where more than one carer is on duty. The wall mounted display indicates clearly the two-digit (or single digit) code representing the setting of the area indicator(s) 7 on the user unit 3 which has transmitted the coded radio signal. In such a case it may not be necessary to provide the LED display on the carer unit itself. The carer unit may be mains operated such that there is sufficient electrical power available to operate a large wall-mounted display.

The carer unit 6 may have an additional button (not shown) which may be operated to show successively the code of all user units which have transmitted a coded radio signal. The carer unit may include a queuing system, which stores any signals received from additional user units after a first user unit has triggered the alarm. On resetting the first user unit, the alarm at the carer unit will continue to sound, and the code of the second user unit will be shown, until the second user unit has been reset, and so on. The LED display 12 may show in rotation the codes of all the user units which have triggered the alarm. Alternatively a button may be operated to scan through all the triggered codes.

The carer unit may have a reset button (not shown) which resets the signal light from red to green.

It should be noted that the user unit may be provided with either one or both of the movement warning system, which detects when the pressure mat circuit is opened, and the pressure control system, which detects when the pressure mat circuit remains closed for a continuous predetermined period of time.

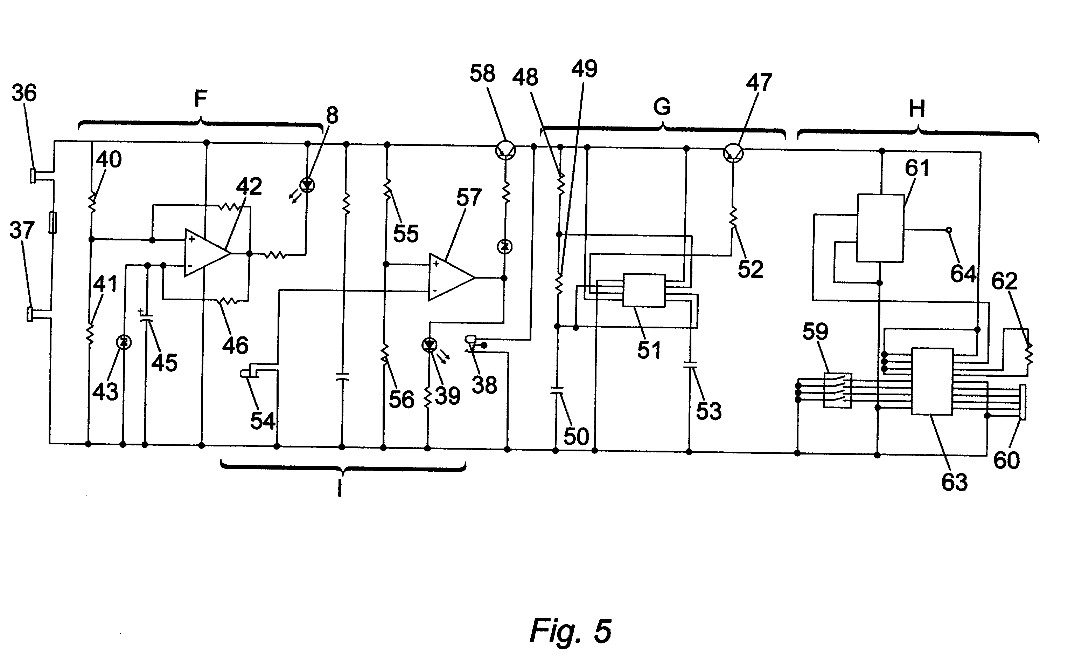

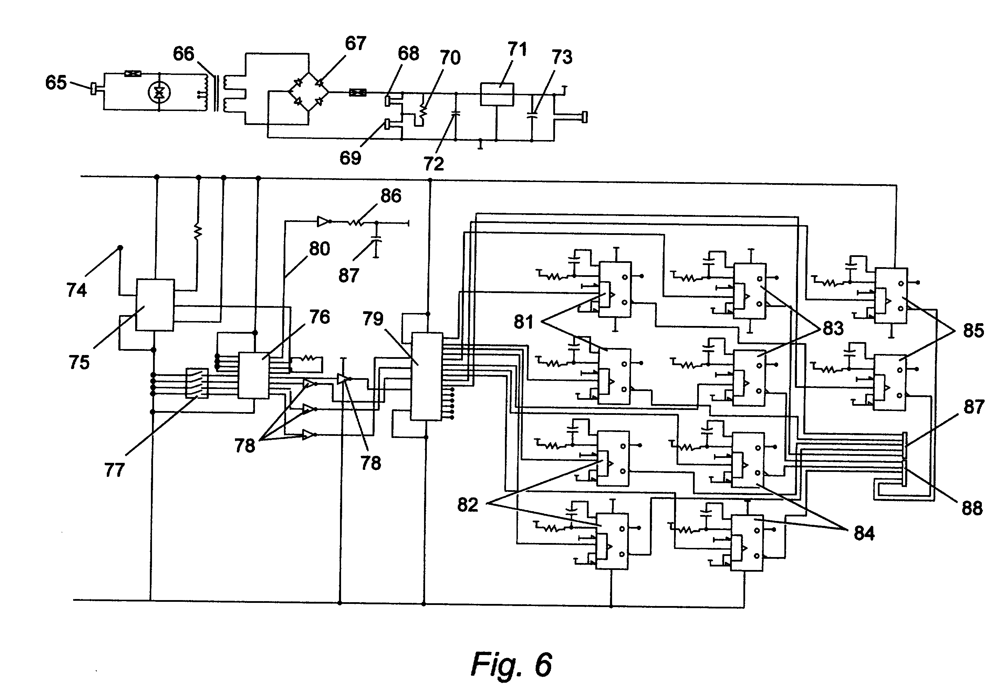

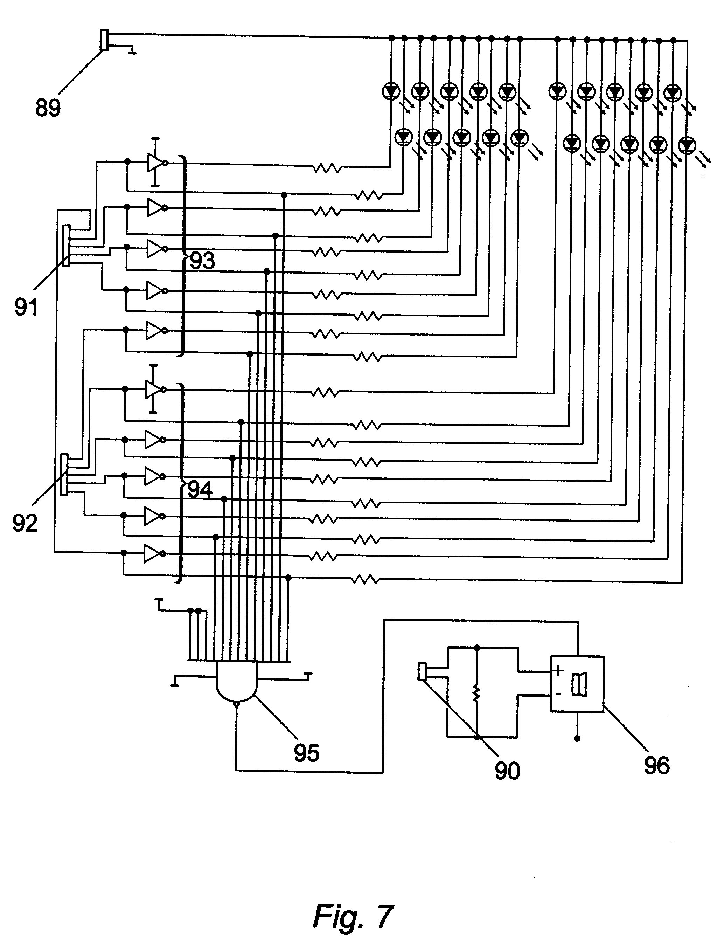

Fig. 5 shows a circuit diagram for the user unit 3, while Figs. 6 and 7 show circuit diagrams for the carer unit 6.

In Fig. 5, resistors 40,41 supply a proportion of the supply line voltage to operational amplifier 42 which is in turn compared to the voltage reference across the zener diode 43. If the proportion of supply voltage falls below the reference, then the output of operational amplifier 42 changes causing the "Battery Low" LED 8 to flash at a rate dictated by capacitor 45 and resistor 46.

The pressure mat 1 is connected to female connector 54 and acts as a simple switch. Resistors 55 and 56 create a reference voltage at the positive terminal of operational amplifier 57. When the patient is on the pressure mat 1, they effectively short the negative terminal of operational amplifier 57 to ground (0 volts), causing the output of operational amplifier 57 to go high and transistor 58 to switch off. By switching off, transistor 58 deprives the following circuit of any supply voltage with which to operate. When the patient moves off the pressure mat 1, the negative terminal of operational amplifier 57 is pulled-up to the supply voltage via resistor 55, causing the output of operational amplifier 57 to go low (0 volts). This in turn switches on transistor 58 which then allows the supply voltage through to the following circuit stages, causing the unit to transmit a signal.

As pressure is removed from the pressure mat 1, the time-slicing circuitry is activated. Since the system is multi-user, the receiving unit must be able to detect multiple alarm signals simultaneously. To achieve this, the user unit 3 is designed to transmit its signal in small bursts of a few milliseconds, repeating every one second. This allows other units to transmit while the first transmitting unit is in its "quiet" state.

The pulse created by the time-slicing circuitry causes transistor 47 to switch on and off, thereby supplying power to the encoding and transmission circuitry in small bursts, and thus allowing transmission in small bursts. Resistors 48,49 and capacitor 50 are used in conjunction with the timer 51 to set the pulse width and period. In addition, resistor 52 is used as a current-limiting resistor and capacitor 53 is used as a decoupling capacitor.

The encoding and transmission circuitry is comprised of mainly "off-the-shelf" components. Switch 59 represents the four least significant parts of the address that is required by the encoder chip 63. The configuration of switch 59 must be the same as the address switch in the receiving unit for the carer unit 6 to accept an alarm signal from the user unit 3. This allows multiple systems to operate in close proximity without one system responding to another system's alarm signal, and ensures each system is set to a different address.

Header pin connector 60 is connected to the "station number" switch 7 which is set by the user. Both this station number and the address are encoded and converted to a serial data stream which is supplied to the input of the transmitter 61. Resistor 62 is used to set the frequency of the serial data stream from the encoder chip 63. The antenna 64 is connected to the out path of transmitter 61.

Referring now to the circuitry of carer unit 6, as shown in Figs. 6 and 7, the carer unit 6 is powered from a 240 volt mains supply connected at pin connector 65. The voltage is stepped down to 12 volts via transformer 66 and bridge rectifier 67. This 12 volt unregulated supply is used to supply the sounder on the display module (Figure 7) via pin connector 69. Pin connector 68 is connected to a 4k7 log potentiometer which acts as a volume control by trimming the 12 volt supply. Resistor 70 ensures that the lowest volume setting is still audible. Line decoder 71 regulates the mains supply to 5 volts in conjunction with capacitors 72,73.

The alarm signal sent by the user unit 3 is picked-up by the antenna 74. The signal is then received and demodulated by receiver 75 which produces a serial data stream at its output. The decoder 76 converts the data stream into parallel data if the address component of the stream matches the address setting on switch 77. The four data lines are inverted by operational amplifiers 78 and multiplexed by line decoder 79, producing the ten station number output lines. Pin 80 of decoder 76 is used to indicate that the decoder 76 has valid data present at its outputs. This signal is used as a strobe to trigger the display of the station number LEDs 12 on the display module.

Because the transmission of an alarm signal only lasts several milliseconds, "one-shot" monostables 81-85 are used to generate longer pulses (approximately 0.5 seconds) which in turn control the duration that the station number LEDs 12 remain lit for. This duration is set by the resistor and capacitor associated with each "one-shot" chip. Each "one-shot" is triggered by a combination of the station number output line and the strobe signal. Resistor 86 and capacitor 87 act as a delay ensuring that the station number signal changes before the strobe signal triggers the monostables 81-85, ensuring that no false-triggering occurs. The outputs from the monostables 81-85 are connected to the display module via data lines 87,88.

Figure 7 details the circuitry of the display module of carer unit 6. The station number output lines from the base unit (Figure 6) are connected to the main board via connector pins 91,92. Each of these lines are complemented using inverters 93,94 so that both states (high and low) of each line can drive corresponding display LEDs 12.

Under normal conditions, when no signals are being received, all the green LEDs are illuminated. When an alarm signal is received, the corresponding station number line changes state, switching off the green LED and illuminating the red LED, indicating that the patient corresponding to the station number has moved off the pressure mat 1.

Input nand gate 95 monitors the state of all the station number lines. If any line is activated, the output of gate 95 goes high, triggering the sounder 96 causing an audible alarm.

The monitoring system of the invention has the following advantages over prior art monitoring systems:
- it uses known pressure mats which are readily available at low cost;
- the user unit may be battery operated, avoiding the need for trailing wires to mains sockets, which can be dangerous for users of limited mobility;
- it uses a coded radio signal to aid identification of the location of the alarm call;
- it includes a lamp, which is illuminated automatically and provides clear visibility at night at the location of the alarm call;
- the remote carer unit or alarm unit may be battery or mains operated;
- the alarm signal on the carer unit may be an audible alarm and may include a small or large digital display;
- the lightness of the pressure mat means that it is easily portable and may be used in a variety of locations without causing discomfort to the user;
- the mat may be easily moved from one site, e.g. a bed, to another site, e.g. a chair, when the user is moved between the sites;
- the softness and flexibility of the pressure mat means that a user may sit or lie directly on the pressure mat without discomfort, and without the formation of pressure points on the user's body;
- the pressure mat may be provided with a washable cover;
- the small size of the mat means that it can be used in a variety of positions, such as above or beneath a cushion, pillow or mattress;
- the user unit and carer unit are both easily portable;
- the digital display at the carer unit may easily display 100 different area numbers, or may display alphanumeric characters.

The transmitter in the user unit 3 is an FM Radiometrix approved by the DTI and requires no radio operating licence in the UK. The radio message is encoded so that the receiver will only accept messages from the appropriate transmitter. The transmitter may be up to 200 m from the receiver, depending on local conditions.

The user unit 3 may incorporate a delay circuit, so that the alarm is not triggered until a specified period of time of a few seconds has elapsed. In this way the alarm will not be triggered by the user momentarily shifting his or her weight.

These and other modifications and improvements can be incorporated without departing from the scope of the invention.

## Claims

1. A carer's monitoring system comprising:
- a flexible pressure sensing mat having electrical contact surfaces which are brought into contact when sufficient pressure is applied to the mat,
- a control unit coupled to said pressure mat, and
- an alarm unit
whereby the control unit is adapted to transmit a first trigger signal to the alarm unit when pressure is removed from the pressure mat such that the electrical contact surfaces are separated from each other.

2. A carer's monitoring system according to Claim 1, wherein the control unit includes delay means adapted to delay the transmission of the first trigger signal until the electrical contact surfaces are separated from each other for a first predetermined time.

3. A carer's monitoring system according to Claim 2, wherein the control unit further comprises a timing means adapted to measure the continuous elapsed period of time for which the electrical contact surfaces are in contact, the control unit being adapted to transmit a second trigger signal when the elapsed period of time reaches a second predetermined time.

4. A carer's monitoring system according to Claim 2 or 3 wherein the control unit further comprises a time setting control adapted to permit selection of one or both of the first and second predetermined times.

5. A carer's monitoring system according to any preceding Claim, whereby the control unit comprises a radio transmitter and the alarm unit comprises a radio receiver, and whereby the trigger signal is a radio signal.

6. A carer's monitoring system according to any preceding Claim, wherein the control unit has a manually operated alarm switch which when operated causes the control unit to send a third trigger signal to the alarm unit.

7. A carer's monitoring system according to any preceding Claim, whereby the control unit further comprises a location indication means in the form of an input device which is operable to select an alphanumeric code relating to the location in which the control unit is placed.

8. A carer's monitoring system according to Claim 7, whereby a coded signal corresponding to the alphanumeric code is transmitted as part of the trigger signal transmitted to the alarm unit.

9. A carer's monitoring system according to Claim 8, wherein the alarm unit is provided with a display means for displaying a signal associated with the transmitted coded signal.

10. A carer's monitoring system comprising a carer unit and a plurality of user units, the carer unit comprising an alarm and a receiver, each user unit comprising:
- a pressure sensing mat having an electrical circuit means which changes from open to short circuit when sufficient pressure is applied to the mat,
- a control unit coupled to said pressure mat, and
- a transmitter,
whereby the control unit is adapted to transmit a first trigger signal to the alarm unit when pressure is removed from the pressure mat such that the electrical circuit means changes from short circuit to open circuit.

11. A carer's monitoring system according to Claim 10, whereby each user unit further comprises a location indication means in the form of an input device which is operable to select an alphanumeric code associated with the location in which the user unit is being used, a coded signal corresponding to the alphanumeric code being transmitted when the trigger signal is transmitted to the alarm unit, the carer unit being provided with a display means for displaying a signal associated with the transmitted coded signal.

12. A carer's monitoring system according to Claim 11, wherein the display means comprises a plurality of pairs of lights, each pair of lights comprising a first light which illuminates when no trigger signal is sent and a second light which illuminates when a trigger signal is sent.

13. A carer's monitoring system according to Claim 11, wherein each pair of lights corresponds to a different alphanumeric code selectable from the input device of the user unit.

14. A carer's monitoring system according to Claim 11, wherein the display means comprises an alphanumeric display which shows the alphanumeric code associated with the trigger signal.
